# EUROPÄISCHE PATENTSCHRIFT

(11) **EP 0 545 150 B1**
(45) Veröffentlichungstag und Bekanntmachung des Hinweises auf die Patenterteilung: **30.06.1999**
(21) Anmeldenummer: 92119690.3
(22) Anmeldetag: 19.11.1992
(51) Int. Cl.: C07D 201/02, C07D 201/08, C07D 207/267, C07C 209/64

(54) **Verfahren zur Herstellung von N-substituierten Lactamen durch reduktive Aminierung von Dicarbonsäure-Derivaten mit gemischen primärer, sekundärer und tertiärer Amine**
Process for the preparation of N-substituted lactams by reductive amination of dicarboxylic acid derivatives with mixtures of primary, secondary and tertiary amines
Procédé pour la préparation de lactames N-substitués par amination réductive de dérivés d'acides dicarboxyliques avec des mélanges d'amines primaires, secondaires et tertiaires

(30) Priorität: 30.11.1991 DE 4139607
(43) Veröffentlichungstag der Anmeldung: 09.06.1993
(73) Patentinhaber: BASF Aktiengesellschaft, 67063 Ludwigshafen (DE)
(72) Erfinder: Weyer, Hans-Jürgen, Dr., W-6800 Mannheim 1 (DE); Fischer, Rolf, Dr., W-6900 Heidelberg (DE)

(56) Entgegenhaltungen:
- EP-A- 0 044 444
- EP-A- 0 252 242
- DE-A- 1 953 263
- DE-A- 2 200 600
- DE-A- 3 904 083
- DE-C- 609 245
- DE-C- 626 923
- GB-A- 596 006
- GB-A- 2 000 121
- GB-A- 2 025 399
- US-A- 2 112 970
- US-A- 2 192 523
- US-A- 3 109 005
- US-A- 3 726 925
- US-A- 4 645 837
- US-A- 4 731 454
- US-A- 4 851 546
- US-A- 5 002 922
- JOURNAL OF ORGANOMETALLIC CHEMISTRY Bd. 330, Nr. 3, 25. August 1987, Seiten 429 - 435 G. BITSI, G. JENNER 'Solvent effect in the ruthenium catalysed carbonylation of amines.'
- JOURNAL OF THE AMERICAN CHEMICAL SOCIETY. Bd. 107, 1985, GASTON, PA US Seiten 361 - 369 R. B. WILSON, R.M. LAINE 'Transalkylation reaction. Homogeneous catalytic formation of C-N bonds.'
- JOURNAL OF THE AMERICAN CHEMICAL SOCIETY. Bd. 101, 1979, GASTON, PA US Seiten 7429 - 7430 S. MURAHASHI, T. WATANABE 'Palladium catalysed hydrolysis of tertiary amines with water.'
- JOURNAL OF THE AMERICAN CHEMICAL SOCIETY. Bd. 100, 1978, GASTON, PA US Seiten 348 - 350 S. MURAHASHI, T. HIRANO, T. YANO 'Palladium catalysed amine exchange reaction of tertiary amines.'
- ISR. J. CHEM. Bd. 27, Nr. 3, 1986, Seiten 267 - 275 R. M. LAINE ET. AL.
- JOURNAL OF THE AMERICAN CHEMICAL SOCIETY. Bd. 95, Nr. 9, 2. Mai 1973, GASTON, PA US Seiten 3038 - 3039 S. MURAHASHI ET. AL. 'Synthesis of Unsymmetrical Secondary and Tertiary Amines from Amines by Palladium catalysts.'

## Beschreibung

Die vorliegende Erfindung betrifft ein Verfahren zur Herstellung von N-substituierten Lactamen der allgemeinen Formel I 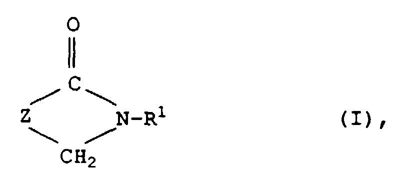 in der
Z für eine C₂- bis C₁₀-Alkylen-, eine C₇- bis C₁₂-Aralkylenlen- oder eine Phenylen- oder Naphthylen-Gruppe steht und R¹ eine C₁- bis C₂₀-Alkyl-, eine C₆- bis C₁₀-Aryl- oder eine C₇- bis C₁₂-Aralkyl-Gruppe ist, durch die hydrierende Umsetzung einer Verbindung der allgemeinen Formel II 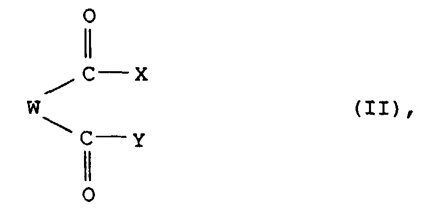 in der
W für eine C₂- bis C₁₀-Alkylen-, eine C₂- bis C₁₀-Alkenylen-, eine C₇- bis C₁₂-Aralkylen- oder eine Phenylen- oder Naphthylen-Gruppe steht und in der die Gruppen X und Y gemeinsam eine Oxa- oder Imido-Brücke der Formeln 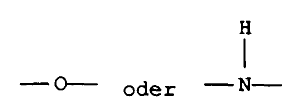 bilden oder alternativ gleich oder verschieden sind und für eine Hydroxylgruppe, eine C₁- bis C₂₀-Alkoxygruppe, eine C₆- bis C₁₀-Aryloxy- oder eine C₇- bis C₁₂-Aralkoxy-Gruppe stehen und in der, falls X und Y verschieden sind, Y außer den obengenannten Bedeutungen noch die Bedeutung Wasserstoff hat bei erhöhtem Druck und bei erhöhter Temperatur und in Gegenwart eines Katalysators und eines Amins.

Lactame können auf verschiedenerlei Weise hergestellt werden. Lactame mit einer größeren Anzahl an Ringatomen, wie ε-Caprolactam, werden beispielsweise über die Beckmann-Umlagerung des betreffenden Oxims erhalten. Zur Herstellung N-substituierter Lactame müssen die so gewonnenen Lactame in einer zweiten Stufe N-alkyliert werden, z.B. durch die Umsetzung mit einem Alkohol. N-substituierte Lactame kleinerer Ringgröße werden im allgemeinen durch die Umsetzung des betreffenden Lactons mit einem primären Amin erhalten.

Neuere Patentanmeldungen beschäftigen sich mit der Herstellung N-substituierter Lactame durch die katalytische Hydrierung von substituierten Dicarbonsäureimiden oder -monoamiden. Diese müssen zuerst durch die Umsetzung von Dicarbonsäurederivaten mit primären Aminen hergestellt werden. Es ist aber auch bekannt, Dicarbonsäuren und deren Derivate in Gegenwart primärer Amine katalytisch zu N-substituierten Lactamen zu hydrieren (vgl. Europäische Patentanmeldung Anmelde-Nr. 91108503).

In US-A 4 731 454 wird die Herstellung von N-Methylpyrrolidon (NMP) durch die Hydrierung von Bernsteinsäure-N-methylimid an einem Kobalt-Rhenium-Molybdän-Katalysator beschrieben. US-A 3 109 005 offenbart ein Verfahren zur Herstellung von NMP durch die Hydrierung von Maleinsäureanhydrid (MSA)-Methylamin-Gemischen an einem Raney-Nickel-Katalysator. Bei Reaktionszeiten von 10 Stunden werden bei diesem Verfahren NMP-Ausbeuten von 70 % erzielt. Gemäß dem Verfahren der DE-A 22 00 600 wird NMP durch die Hydrierung von MSA-Methylamin-Gemischen an Palladium-Trägerkatalysatoren unter den günstigsten Bedingungen in einer Ausbeute von 44 % erhalten.

Alle diese Herstellungsverfahren haben den Nachteil, daß zu ihrer Durchführung reine primäre Amine benötigt werden. Bei der Herstellung von primären Aminen fallen diese insbesondere bei großtechnischen Herstellungsverfahren jedoch nicht in reiner Form an, sondern als Gemische aus primären, sekundären und tertiären Aminen. Somit müssen bislang zur Herstellung von N-substituierten Lactamen durch die hydrierende Aminierung von Dicarbonsäuren oder Dicarbonsäurederivaten, wie Dicarbonsäureanhydriden oder -estern, die hierzu benötigten primären Amine, in aufwendigen Reinigungsverfahren von tertiären und sekundären Aminen abgetrennt werden. Durch dieses Erfordernis werden diese Verfahren erheblich verteuert.

Der vorliegenden Erfindung lag somit die Aufgabe zugrunde, ein wirtschaftlich arbeitendes Verfahren zur finden, daß die Verwendung von sekundären und tertiären Aminen als Aminquelle bei der hydrierenden Aminierung von Dicarbonsäuren, Dicarbonsäurederivaten und/oder Formylcarbonsäuren und/oder deren Derivaten zu N-substituierten Lactamen ermöglicht.

Dementsprechend wurde ein Verfahren zur Herstellung von N-substituierten Lactamen der allgemeinen Formel I 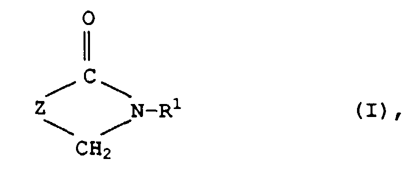 in der Z für eine C₂- bis C₁₀-Alkylen-, eine C₇- bis C₁₂-Aralkylenkylen- oder eine Phenylen- oder Naphthylen-Gruppe steht und R¹ eine C₁- bis C₂₀-Alkyl-, eine C₆- bis C₁₀-Aryl- oder eine C₇- bis C₁₂-Aralkyl-Gruppe ist, durch die hydrierende Umsetzung einer Verbindung der allgemeinen Formel II 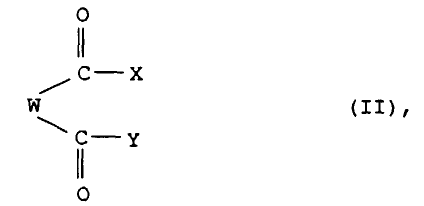 in der W für eine C₂- bis C₁₀-Alkylen-, eine C₂- bis C₁₀-Alkenylen-, eine C₇- bis C₁₂-Aralkylen- oder eine Phenylen- oder Naphthylen-Gruppe steht und in der die Gruppen X und Y gemeinsam eine Oxa- oder Imido-Brücke der Formeln 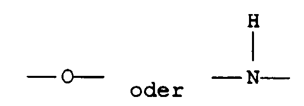 bilden oder alternativ gleich oder verschieden sind und für eine Hydroxylgruppe, eine C₁- bis C₂₀-Alkoxygruppe, eine C₆- bis C₁₀-Aryloxy- oder eine C₇- bis C₁₂-Aralkoxy-Gruppe stehen und in der, falls X und Y verschieden sind, Y außer den obengenannten Bedeutungen noch die Bedeutung Wasserstoff hat, bei erhöhtem Druck und bei erhöhter Temperatur in Gegenwart eines Katalysators und eines Amins gefunden, das dadurch gekennzeichnet ist, daß man sekundäre und/oder tertiäre Amine der Formel III

NHₙR¹ ₃₋ₙ (III)

in der der Rest R¹ die obengenannte Bedeutung hat und in der der Index n für 0 oder 1 steht, oder Mischungen dieser sekundären und/oder tertiären Amine mit einem primären Amin der Formel IV

R¹-NH₂ (IV)

als Ausgangsmaterial benutzt und die Umsetzung unter Zusatz von Wasser und/oder Ammoniak durchführt.

Das erfindungsgemäße Verfahren ist somit in der Lage, sowohl sekundäre als auch tertiäre Amine oder Gemische dieser Amine als Aminquelle bei der Herstellung von N-substituierten Lactamen zu nutzen. Besonders bevorzugt werden aber Amingemische im erfindungsgemäßen Verfahren eingesetzt, wie sie direkt bei der reduktiven Aminierung von Alkoholen, beispielsweise gemäß den Verfahren von DE-A 19 53 263 und US-A 5 002 922, anfallen. Die Verwendung derartiger Amingemische, welche primäre, sekundäre und tertiäre Amine enthalten, hat den Vorteil, daß keine zusätzliche Reinigungsstufe zur Isolierung einer bestimmten Aminspezies aus dem Amingemisch der reduktiven Aminierung mehr erforderlich ist. Der Gehalt derartiger Amingemische an den einzelnen primären, sekundären und tertiären Aminkomponenten ist für das erfindungsgemäße Verfahren im allgemeinen nicht kritisch, d.h. es können sowohl Amingemische eingesetzt werden, die überwiegend primäre und wenig sekundäre und/oder tertiäre Amine enthalten als auch Amingemische, welche keine primäre Amine enthalten.

Die Amine werden bezüglich der Dicarbonsäuren, Dicarbonsäurederivate, Formylcarbonsäuren und/oder Formylcarbonsäurederivate II üblicherweise in einem Molverhältnis Amin/Verbindung II von 0,5:1 bis 5:1, vorzugsweise von 0,8:1 bis 2:1 und besonders bevorzugt in einem Molverhältnis von 1:1 bis 1,5:1 eingesetzt. Selbstverständlich können auch größere oder kleinere Molverhältnisse angewandt werden, doch sind diese in der Regel aus wirtschaftlichen Gründen weniger bevorzugt. Werden Amingemische im erfindungsgemäßen Verfahren benutzt, so wird zur Berechnung des Molverhältnisses Amin/Verbindung II die Summe der Mole der im Amingemisch enthaltenen primären, sekundären und tertiären Aminkomponenten herangezogen.

Für den Erfolg des erfindungsgemäßen Verfahrens ist es wichtig, daß dem Reaktionsgemisch Wasser und/oder Ammoniak zugefügt wird. Das Wasser sollte im Reaktionsgemisch in frei verfügbarer Form vorliegen, da es vermutlich an der Umsetzung teilnimmt. Im allgemeinen wird das Wasser bezüglich der im Reaktionsgemisch enthaltenen Amine in einer Menge von 0,1 bis 100, vorzugsweise von 1 bis 50 und insbesondere von 5 bis 20 Mol/pro Mol Amin oder Amine zugegeben.

Die Anwendung von Ammoniak anstelle von Wasser kann im erfindungsgemäßen Verfahren insbesondere dann besonders vorteilhaft sein, wenn die Umsetzung der Dicarbonsäuren oder Dicarbonsäurederivate in einem nichtwäßrigen Medium durchgeführt werden soll. Eine wasserfreie Arbeitsweise kann insbesondere dann zweckmäßig sein, wenn sich die Reaktionspartner nicht in ausreichendem Maße in Wasser lösen. Das Ammoniak wird in der Regel in Mengen von 0,1 bis 100, bevorzugt von 1 bis 50 und besonders vorteilhaft in Mengen von 1 bis 10 Mol/pro Mol Amin oder Amine eingesetzt.

Selbstverständlich können im erfindungsgemäßen Verfahren auch wäßrige Ammoniaklösungen vorteilhaft angewandt werden. Im Hinblick auf die optimale Dosierung des Wassers und/oder des Ammoniaks im Rahmen der oben angegebenen Bereiche ist noch anzufügen, daß diese vorteilhafterweise für die jeweils angewandte Aminmischung in einfachen Vorversuchen ermittelt werden kann.

Die erfindungsgemäße Umsetzung kann in An- oder Abwesenheit eines Lösungsmittels durchgeführt werden. Als Lösungsmittel können praktisch alle Lösungsmittel verwendet werden, die sich unter den Reaktionsbedingungen inert verhalten, z.B. Wasser, aliphatische und aromatische Kohlenwasserstoffe, wie Pentan, Hexan, Heptan, Octan, Benzol, Toluol oder Xylol, Ether, wie Diethylether, Diisopropylether, Methyl-tert.-butylether, Dioxan oder Tetrahydrofuran oder Gemische dieser Lösungsmittel, Einsatz finden. Vorteilhaft können auch N-substituierte Lactame, insbesondere dasjenige Lactam, das bei der betreffenden Umsetzung als Produkt entsteht, als Lösungsmittel benutzt werden. Besonders bevorzugt ist das Lösungsmittel Wasser. Die Verbindungen II werden im allgemeinen mit dem Lösungsmittel in einem Molverhältnis Verbindung II/Lösungsmittel von 1:1 bis 1:100, vornehmlich von 1:5 bis 1:50 gemischt.

Der zur Umsetzung benötigte Wasserstoff kann in stöchiometrischen Mengen zur Reaktionsmischung gegeben werden, zweckmäßigerweise wird mit einem Überschuß an Wasserstoff gearbeitet. Die Höhe des im erfindungsgemäßen Verfahren angewandten Wasserstoffüberschußes ist nicht kritisch, da der unverbrauchte Wasserstoff wieder in die Umsetzung zurückgeführt werden kann. Der Wasserstoff kann in reiner Form, als auch mit einem Inertgas, wie Stickstoff oder Argon, verdünnt der Umsetzung zugeführt werden; bevorzugt wird unverdünnter Wasserstoff verwendet.

Die hydrierende Umsetzung der Verbindungen II mit den sekundären oder tertiären Aminen oder den diese Amine enthaltenden Amingemischen erfolgt üblicherweise bei Temperaturen von 100 bis 350°C, vorzugsweise bei 150 bis 300°C und bei einem Druck von 50 bis 350 bar, insbesondere von 100 bis 300 bar. Der bei den jeweiligen Reaktionstemperaturen gewünschte Druck im Reaktor kann zweckmäßigerweise durch Einpressen von Wasserstoff in den Reaktor eingestellt werden. Die Zugabereihenfolge der Reaktanten in den Reaktor ist im allgemeinen nicht kritisch.

Die Umsetzung kann diskontinuierlich, beispielsweise in Rührautoklaven, durchgeführt werden, vorzugsweise wird aber die kontinuierliche Fahrweise, z.B. in Rohrreaktoren oder Rohrbündelreaktoren, gewählt, wobei die Hydrierwärme durch Außen- oder Innenkühlung abgeführt werden kann. Eine weitere Möglichkeit zur Regulierung der Reaktionstemperatur besteht in der Rückführung eines Teils des Hydrieraustrages sowie des überschüssigen Wasserstoffs nach vorhergehender Abkühlung, beispielsweise in einem Wärmetauscher.

Die hydrierende Umsetzung kann so gesteuert werden, daß während der gesamten Umsetzungsdauer ein bestimmter Druck- und Temperaturbereich eingehalten wird. Es kann sich aber, insbesondere beim Durchsatz größerer Mengen, positiv für die Selektivität und die Standzeit des Katalysators auswirken, wenn man die Umsetzung auf verschiedenen Druck- und Temperaturniveaus durchführt, beispielsweise indem man zunächst in einem ersten Reaktor bei Temperaturen von 100 bis 220°C und Drucken von 50 bis 200 bar teilhydriert und anschließend den Hydrieraustrag ohne Aufarbeitung in den nächsten Reaktor leitet, um dort die hydrierende Umsetzung z.B. bei Temperaturen von 220 bis 300°C und Drucken von 200 bis 350 bar zu Ende zu führen.

Die so erhaltenen Hydrierausträge können unter Umständen neben den erwünschten N-substituierten Lactamen noch geringe Mengen an Nebenprodukten, wie N-substituierte Dicarbonsäureimide, Dicarbonsäurediamide, Dicarbonsäuremonoamide, u.ä. enthalten. Die Aufarbeitung dieser Hydrierausträge kann durch Extraktion oder vorteilhaft destillativ erfolgen. Die so abgetrennten, teilhydrierten Nebenprodukte können, da sie zu N-substituierten Lactamen hydrierbar sind, zur Erzielung eines vollständigen Umsatzes in die Hydrierzone zurückgeführt werden.

Zur hydrierenden Umsetzung können an sich übliche, heterogene Hydrierkatalysatoren eingesetzt werden, insbesondere solche, welche in ihrer aktiven Masse mindestens ein Element der ersten, siebten oder achten Nebengruppe des Periodensystems der Elemente, insbesondere mindestens eines der Elemente Eisen, Rhenium, Ruthenium, Kobalt, Rhodium, Nickel, Palladium, Platin, Kupfer oder Silber enthalten. Als weitere Elemente, die in der katalytisch aktiven Masse enthalten sein können, seien Chrom, Molybdän, Wolfram und/oder Mangan beispielhaft genannt. Die Katalysatoren können in fein verteilter Form, z.B. als Raney-Nickel, Raney-Kobalt, Platinschwamm, Palladiumschwamm oder Carbonyleisenpulver oder aber auch in Form von Spänen, Netzen oder sonstigen Gebilden großer Oberfläche eingesetzt werden. Es ist auch vorteilhaft Trägerkatalysatoren, welche die katalytisch aktiven Metalle auf einem inerten Trägermaterial, wie Aktivkohle, Siliziumdioxid, Aluminiumoxid, Titandioxid, Zirkondioxid, auf einem Silikat, wie Steatit oder Bimsstein, auf Bariumsulfat oder Calciumcarbonat, abgeschieden enthalten, zu verwenden. Als solche Katalysatoren seien beispielhaft Palladium auf Aktivkohle, Platin auf Aktivkohle, Palladium auf Bariumsulfat oder Palladium auf Calciumcarbonat aufgezählt.

Fällungskatalysatoren können ebenfalls mit sehr guten Ergebnissen im erfindungsgemäßen Verfahren angewandt werden. Unter Fällungskatalysatoren werden solche Katalysatoren verstanden, deren katalytisch aktive Komponenten aus den Lösungen ihrer Salze, beispielsweise ihre Nitrate, mit Hilfe eines Fällungsmittels, z.B. mittels einer Alkalimetall- oder Erdalkalimetall-Hydroxidlösung oder einer Alkalimetall- oder Erdalkalimetall-Carbonatlösung, ausgefällt und anschließend getrocknet und calciniert werden. Die so erhaltenen calcinierten Katalysatormassen, welche die katalytisch aktiven Komponenten, hauptsächlich in oxidischer Form enthalten, können gewünschtenfalls mit Hilfe eines Formhilfsmittels, wie Graphit, Stearinsäure oder Phosphorsäure verformt werden. Vor ihrem Einsatz als Katalysatoren werden die so erhaltenen Katalysatorformkörper in der Regel durch die Reduktion im Wasserstoffstrom bei erhöhter Temperatur aktiviert, wobei die mittels Wasserstoff reduzierbaren Katalysatorkomponenten reduziert werden, in der Regel größtenteils zu den betreffenden Metallen. Diese Fällungskatalysatoren können in kompakter Form, d.h. ohne Trägermaterialien, hergestellt werden, sie können vorteilhaft aber auch als Trägerkatalysatoren erhalten werden. Zu diesem Zweck können die katalytisch aktiven Komponenten beispielsweise auf ein vorgefälltes Trägermaterial aufgefällt werden oder gemeinsam mit dem Trägermaterial in einer Mischfällung aus einer Lösung der betreffenden Salze ausgefällt werden. Als Trägermaterialien fur derartige Fällungskatalysatoren sind z.B. Siliziumdioxid, Aluminiumoxide, Titandioxide, Zirkondioxid oder Silikate besonders geeignet.

Besonders bevorzugt werden im erfindungsgemäßen Verfahren außer den zuvor erwähnten Katalysatoren solche Katalysatoren verwendet, wie sie in US-A 4 731 454, DE-A 23 21 101, DE-A 39 04 083, EP-A 44 444 und EP-A 147 219 beschrieben sind, verwendet.

Die Katalysatoren können im erfindungsgemäßen Verfahren in suspendierter Form angewandt werden, bevorzugt ist jedoch eine Festbettanordnung des Katalysators, über die die Einsatzstoffe in der Sumpf- oder vorzugsweise in der Rieselfahrweise geleitet werden können.

Als Ausgangsmaterial zur Herstellung von N-substituierten Lactamen nach dem erfindungsgemäßen Verfahren dienen die Verbindungen der allgemeinen Formel II 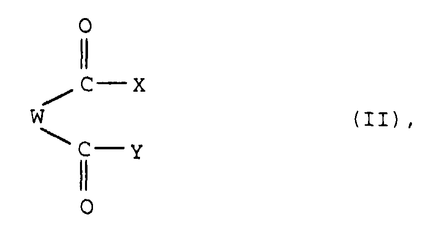 in der W eine C₂- bis C₁₀-Alkylen-, eine C₂- bis C₁₀-Alkenylen-, eine C₇- bis C₁₂-Aralkylen- oder eine Phenylen- oder Naphthylen-Gruppe sein kann und in der die Gruppen X und Y gemeinsam eine Oxa- oder Imido-Brücke der Formeln bilden können, oder alternativ gleich oder verschieden sein können und für eine Hydroxylgruppe, eine C₁- bis C₂₀-Alkoxygruppe, eine C₆- bis C₁₀-Aryloxy- oder eine C₇- bis C₁₂-Aralkoxygruppe stehen können und in der, falls X und Y verschieden sind, Y außer den obengenannten Bedeutungen noch die Bedeutung Wasserstoff haben kann.

Vorzugsweise werden Verbindungen II eingesetzt, in denen die Gruppe Z eine C₂- bis C₄-Alkylen-, eine C₂- bis C₄-Alkenylenlen- oder eine Phenylen-Gruppe ist. Die Alkylen- und Alkenylen-Gruppen Z können geradkettig, verzweigt oder cyclisch sein. Besonders bevorzugte Gruppen Z sind die Ethylen-, Ethenylen-, 1,3-Propylen-, 1,2-Propylen-, 1-Propen-3-yl-, 1-Propen-2-yl-, 1,4-Butylen- sowie die 1,2-Phenylen-Gruppe.

Die Gruppen X und Y im Ausgangsmaterial der Formel II können gleich oder verschieden sein. Beispielsweise können X und Y gemeinsam eine Oxa- oder Imido-Brücke ausbilden, so daß die Verbindungen II cyclische Dicarbonsäureanhydride oder -imide verkörpern. Die Ausgangsverbindungen II können aber auch offenkettig und Dicarbonsäuren, falls X und Y für eine Hydroxylgruppe stehen, oder Dicarbonsäurediester falls X und Y für eine Alkoxy-, Aryloxy- und/oder eine Aralkoxy-Gruppe stehen, sein.

Selbstverständlich können auch solche Ausgangsverbindungen II im erfindungsgemäßen Verfahren erfolgreich verwendet werden, in denen die Gruppen X und Y verschieden sind und für eine der obengenannten funktionellen Gruppen stehen. Als solche Verbindungen seien z.B. Dicarbonsäuremonoester, Formylcarbonsäuren und Formylcarbonsäureester genannt. Der Verwendung der Formylcarbonsäuren der Formel IIa 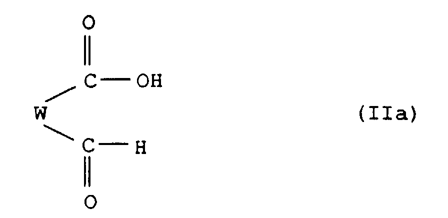 ist selbstverständlich die Verwendung von deren Lactolen der Formel V 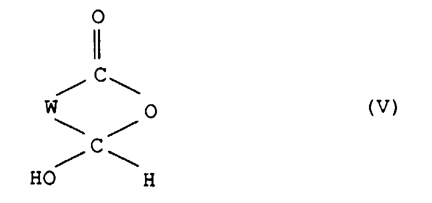 äquivalent, da die Formylcarbonsäuren IIa in Gegenwart von Wasser mit ihren Lactolen V im Gleichgewicht stehen.

Das gleiche gilt für Mischungen der verschiedenen Dicarbonsäurederivate. Gleichwohl ist im allgemeinen die Verwendung solcher Ausgangsverbindungen II bevorzugt, in denen die Gruppen X und Y gleich sind. Ebenso sind solche Formylcarbonsäureester bevorzugte Ausgangsmaterialien, die kostengünstig durch die Hydroformylierung der betreffenden Alkencarbonsäurester, z.B. durch die Hydroformylierung der Acryl- oder Methacrylsäureester nach dem Verfahren von EP-A 173 226, zur Verfügung gestellt werden können.

Bevorzugte Gruppen X und Y sind die Hydroxylgruppe, C₁- bis C₁₀-Alkoxy-, insbesondere C₁- bis C₄-Alkoxygruppen, wie die Methoxy-, Ethoxy-, Propoxy-, Isopropoxy-Gruppe sowie die Butoxygruppen, die Phenoxygruppe und die Benzyloxygruppe. Y steht zudem bevorzugt für Wasserstoff.

Besonders bevorzugte Ausgangsmaterialien sind Maleinsäure, Maleinsäureanhydrid, Maleinsäureimid, Maleinsäure-C₁- bis C₄-dialkylester, Bernsteinsäure, Bernsteinsäureanhydrid, Bernsteinsäureimid, Bernsteinsäure-C₁- bis C₄-dialkylester, Adipinsäure, Adipinsäure-C₁- bis C₄-dialkylester, Phthalsäure, Phthalsäureanhydrid, Phthalsäureimid sowie Phthalsäure-C₁- bis C₁₀-dialkylester, Maleinsäure-C₁- bis C₄-monoalkylester, Bernsteinsäure-C₁- bis C₄-monoalkylester, Adipinsäure-C₁- bis C₄-monoalkylester, Phthalsäure-C₁- bis C₄-monoalkylester, 3-Formyl-C₁- bis C₄-propionsäureester und 3-Formyl-2-methyl-C₁- bis C₄-propionsäureester.

Die Ausgangsverbindungen II sind großtechnische Produkte und stehen in großen Mengen zur Verfügung. Maleinsäureanhydrid kann durch die Oxidation von Aromaten, Buten oder Butan nach den in K. Weissermel, H.-J. Arpe: Industrielle organische Chemie, 2. Auflage, Seite 343 bis 349, Verlag Chemie, Weinheim 1978, geschilderten Methoden hergestellt werden. Die Hydrolyse bzw. Alkoholyse des Maleinsäureanhydrids führt zu Maleinsäure bzw. Maleinsäuremonoestern. Maleinsäurediester können daraus in einer herkömmlichen Veresterungsreaktion erhalten werden. Bernsteinsäure und die genannten Bernsteinsäurederivate können aus Maleinsäure und den entsprechenden Maleinsäurederivaten durch die Hydrierung der Doppelbindung erhalten werden. Cyclische Dicarbonsäureimide können nach den Verfahren von Chem. Rev. 70, 439 (1970) hergestellt werden. Adipinsäure wird großtechnisch durch die Oxidation von Cyclohexanol/Cyclohexanon hergestellt (vgl. Weissermel, Arpe: Seite 227 bis 229). Die Adipinsäureester können daraus nach herkömmlichen Veresterungsverfahren erzeugt werden. Phthalsäureanhydrid wird großtechnisch durch die Oxidation von Xylol produziert und ebenfalls großtechnisch nach herkömmlichen Verfahren zu Phthalsäureestern umgesetzt (vgl. Weissermel, Arpe: Seite 359 bis 364). Die Hydrolyse des Phthalsäureanhydrids ergibt Phthalsäure, seine Alkoholyse Phthalsäuremonoester.

Das erfindungsgemäße Verfahren zur Herstellung N-substituierter Lactame kann sekundäre und tertiäre Amine der Formel III

NHₙR¹ ₃₋ₙ (III)

als Aminquelle nutzen, in denen der Rest R¹ für eine C₂- bis C₁₀-Alkyl-, eine C₆- bis C₁₀-Aryl- und/oder eine C₇- bis C₁₂-Aralkylgruppe steht und in der der Index n 0 oder 1 bedeutet. Die aliphatischen Reste R¹ können geradkettig, verzweigt oder cyclisch sein. Besonders bevorzugte Reste R¹ sind die Methyl-, Ethyl-, Propyl- und die n-Butyl-Gruppe. Wie bereits erwähnt, können diese Amine durch die hydrierende Aminierung der entsprechenden Alkohole und Ketone erzeugt werden.

Bei der erfindungsgemäßen Umsetzung können sich unter den angewandten Reaktionsbedingungen aus den Carbonsäureverbindungen II und den erfindungsgemäß verwendeten sekundären und tertiären Aminen III intermediär die entsprechenden Dicarbonsäurediamide, Dicarbonsäuremonoamide, Formylcarbonsäureamide, Formylcarbonsäureammoniumsalze, Dicarbonsäure-mono- und -di-ammoniumsalze, Dicarbonsäure-monoester-monoamide, Dicarbonsäure-monoamid-monoammoniumsalze u.ä. bilden, welche glatt zu den gewünschten Lactamen weiterreagieren. Es versteht sich daher von selbst, daß anstelle der bereits erwähnten Ausgangsmaterialien auch diese Intermediate eingesetzt werden können und daß deren Verwendung dem Einsatz der zuvor erwähnten Ausgangsmaterialien äquivalent ist.

Für die nach dem erfindungsgemäßen Verfahren herstellbaren N-substituierten Lactame gibt es eine Vielzahl von Verwendungen, z.B. als polare Lösungs- oder Extraktionsmittel. Beispielsweise wird N-Methylpyrrolidon als Lösungsmittel für Polymere, wie Polyurethane, Polyimide, Polyamide und Polyarylensulfide, als Extraktionsmittel für Acetylen, Butadien und aromatische Kohlenwasserstoffe sowie als Lösungsmittel für chemische Umsetzungen benutzt (vgl. hierzu: Ullmanns Encyklopädie der technischen Chemie, 4. Auflage, Band 19, Seite 641 bis 642, Verlag Chemie, Weinheim 1980).

### Beispiele

In den folgenden Beispielen wurden Katalysatoren, die im nichtreduzierten Zustand die folgende Zusammensetzung hatten, verwendet:
Katalysator A (gemäß EP-A 44 444)
   50 Gew.-% Cu, berechnet als CuO
   50 Gew.-% Al, berechnet als Al₂O₃
Katalysator B (gemäß DE-A 39 04 083)
   63,4 Gew.-% Co, berechnet als CoO
   18,1 Gew.-% Cu, berechnet als CuO
   6,8 Gew.-% Mn, berechnet als Mn₂O₃
   3,1 Gew.-% Mo, berechnet als MoO₃
   0,15 Gew.-% Na, berechnet als Na₂O
   3,3 Gew.-% Phosphorsäure, berechnet als H₃PO₄

### Beispiel 1

In einen mit einem Begasungsrührer ausgestatteten Autoklaven wurden 9,8 g (0,1 mol) MSA, 100 g Wasser, 0,4 mol Amin und 10 g Katalysator A als Suspensionskatalysator gegeben. Anschließend wurde der Autoklav auf 225°C erhitzt und Wasserstoff mit einem Druck von 200 bar in den Autoklaven gepreßt. Als keine Wasserstoffaufnahme mehr beobachtet werden konnte, wurde die Reaktion abgebrochen und der Autoklaveninhalt gaschromatographisch analysiert. Für die bei den einzelnen Umsetzungen eingesetzten Amine wurden die in Tabelle 1 aufgeführten Ausbeuten an NMP erzielt.

**Tabelle 1**

| Eingesetztes Amin | NMP-Ausbeute (bez. auf eingesetztes MSA) |
|---|---|
| Dimethylamin | 53 % |
| Trimethylamin | 41 % |
| Monomethylamin | 38 % |

### Beispiel 2

In einen beheizbaren Rohrreaktor (Länge 200 mm, Innendurchmesser 16 mm) in dem 38 g Katalysator B in einem Festbett angeordnet waren, wurden die Reaktanten in Rieselfahrweise bei einer Temperatur von 250°C und einem Gesamtdruck von 200 bar über den Katalysator geleitet und filtriert. Der Reaktionsaustrag wurde auf Raumtemperatur abgekühlt, entspannt und in einem Gas-Flüssigkeits-Abscheider in seine gasförmigen und flüssigen Bestandteile zerlegt. Die Zusammensetzung des erhaltenen Produktgemisches wurde gaschromatographisch bestimmt.

Bei dieser Umsetzung wurde der Katalysator stündlich mit jeweils 0,15 kg (1,5 mol) MSA, 0,06 kg (0,94 mol) Monomethylamin, 0,02 kg (0,44 mol) Dimethylamin, 0,01 kg (0,17 mol) Trimethylamin und 0,55 kg Wasser pro kg Katalysator belastet, wobei stündlich 2500 NL Wasserstoff pro kg Katalysator zugeführt wurden.

Unter diesen Bedingungen wurde während einer Laufzeit von 9 Stunden eine durchschnittliche NMP-Ausbeute von 89 %, bezogen auf das eingesetzte MSA, erzielt. Wurde der Katalysator unter denselben Reaktionsbedingungen hingegen nur mit 0,06 kg Monomethylamin belastet, wurde nur eine NMP-Ausbeute von 77 %, bezogen auf eingesetztes MSA, erhalten.

### Beispiel 3

In einen mit einem Begasungsrührer ausgestatteten Autoklaven wurden 5 g (0,05 mol) MSA, 100 g Tetrahydrofuran, 9 g (0,06 mol) Dicyclohexylamin, 4 g Ammoniak und 10 g Katalysator B gegeben. Anschließend wurde der Autoklav auf 225°C erhitzt und Wasserstoff mit einem Druck von 200 bar in den Autoklaven gepreßt. Als keine Wasserstoffaufnahme mehr beobachtet wurde, wurde die Reaktion abgebrochen. Die gaschromatographische Untersuchung der Reaktionsmischung ergab eine Ausbeute von 8,8 % N-Cyclohexylpyrrolidon.

## Patentansprüche

1. Verfahren zur Herstellung von N-substituierten Lactamen der allgemeinen Formel I 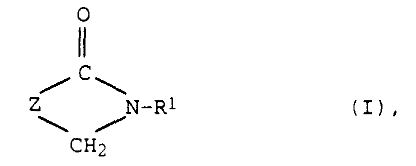 in der
Z für eine C₂- bis C₁₀-Alkylen-, eine C₇- bis C₁₂-Aralkylenlen- oder eine Phenylen- oder Naphthylen-Gruppe steht und
R¹ eine C₁- bis C₂₀-Alkyl-, eine C₆- bis C₁₀-Aryl- oder eine C₇- bis C₁₂-Aralkyl-Gruppe ist, durch die hydrierende Umsetzung einer Verbindung der allgemeinen Formel II 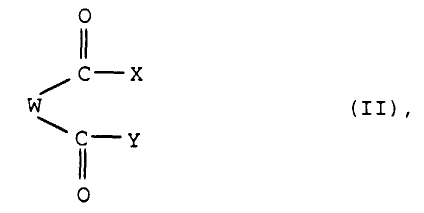
in der
W für eine C₂- bis C₁₀-Alkylen-, eine C₂- bis C₁₀-Alkenylen-, eine C₇- bis C₁₂-Aralkylen- oder eine Phenylen- oder Naphthylen-Gruppe steht und in der die Gruppen X und Y gemeinsam eine Oxa- oder Imido-Brücke der Formeln 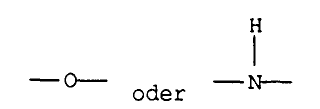 bilden oder alternativ gleich oder verschieden sind und für eine Hydroxylgruppe, eine C₁- bis C₂₀-Alkoxygruppe, eine C₆- bis C₁₀-Aryloxy- und/oder eine C₇- bis C₁₂-Aralkoxy-Gruppe stehen und in der, falls X und Y verschieden sind, Y außer den obengenannten Bedeutungen noch die Bedeutung Wasserstoff hat, bei erhöhtem Druck und bei erhöhter Temperatur in Gegenwart eines Katalysators und eines Amins, dadurch gekennzeichnet, daß man sekundäre und/oder tertiäre Amine der Formel III
NHₙR¹ ₃₋ₙ (III)
in der der Rest R¹ die obengenannte Bedeutung hat und der Index n für 0 oder 1 steht, oder Mischungen dieser sekundären und/oder tertiären Amine mit einem primären Amin der Formel IV
R¹-NH₂ (IV)
als Ausgangsmaterial benutzt und die Umsetzung unter Zusatz von Wasser und/oder Ammoniak durchführt.

2. Verfahren nach Anspruch 1, dadurch gekennzeichnet, daß man die Umsetzung in Gegenwart von mindestens 1 mol Wasser pro Mol eingesetzter Amine durchführt.

3. Verfahren nach Anspruch 1, dadurch gekennzeichnet, daß man die Umsetzung in Gegenwart von mindestens 1 mol Ammoniak pro Mol eingesetzter Amine durchführt.

4. Verfahren nach Anspruch 1, dadurch gekennzeichnet, daß man die Umsetzung in einem Lösungsmittel durchführt.

5. Verfahren nach Anspruch 1, dadurch gekennzeichnet, daß man die Umsetzung in Wasser als Lösungsmittel vornimmt.

6. Verfahren nach Anspruch 1, dadurch gekennzeichnet, daß man bei einer Temperatur von 100 bis 300°C und bei einem Druck von 50 bis 350 bar arbeitet.

7. Verfahren nach Anspruch 1, dadurch gekennzeichnet, daß man einen Katalysator verwendet, dessen aktive Komponente mindestens ein Element der ersten, siebten oder achten Nebengruppe des Periodensystems der Elemente enthält.

## Claims

1. A process for the preparation of N-substituted lactams of the formula I 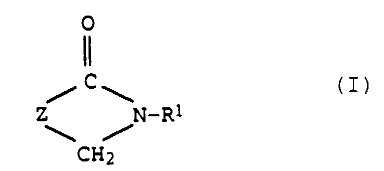 where
Z is C₂- to C₁₀-alkylene, C₇- to C₁₂-aralkylene, phenylene or naphthylene, and
R¹ is C₁- to C₂₀-alkyl, C₆- to C₁₀-aryl or C₇- to C₁₂-aralkyl,
by hydrogenating a compound of the formula II 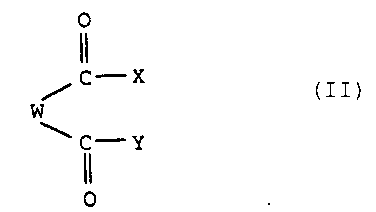 where
W is C₂- to C₁₀-alkylene, C₂- to C₁₀-alkenylene, C₇- to C₁₂-aralkylene, phenylene or naphthylene, and
X and Y together form an oxa or imido bridge of the formula 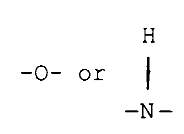
or alternatively are identical or different and are hydroxyl, C₁- to C₂₀-alkoxy, C₆- to C₁₀-aryloxy and/or C₇- to C₁₂-aralkoxy, and, if X and Y are different, Y, in addition to the abovementioned meanings, may also be hydrogen,
at superatmospheric pressure and at elevated temperature in the presence of a catalyst and in the presence of an amine, which comprises using a secondary and/or tertiary amine of the formula III
NHₙR¹ ₃₋ₙ (III)
where R¹ is as defined above and n is 0 or 1, or a mixture of a secondary and/or tertiary amine of this type with a primary amine of the formula IV
R¹-NH₂ (IV)
as the starting material, and carrying out the reaction with addition of water and/or ammonia.

2. A process as claimed in claim 1, wherein the reaction is carried out in the presence of at least 1 mol of water per mole of amine.

3. A process as claimed in claim 1, wherein the reaction is carried out in the presence of at least 1 mol of ammonia per mole of amine.

4. A process as claimed in claim 1, wherein the reaction is carried out in a solvent.

5. A process as claimed in claim 1, wherein the reaction is carried out in water as solvent.

6. A process as claimed in claim 1, wherein the reaction is carried out at from 100 to 300°C and at from 50 to 350 bar.

7. A process as claimed in claim 1, wherein a catalyst is used whose active component contains at least one element of the first, seventh or eighth subgroup of the Periodic Table of the Elements.

## Revendications

1. Procédé de préparation de lactames N-substitués de formule générale I 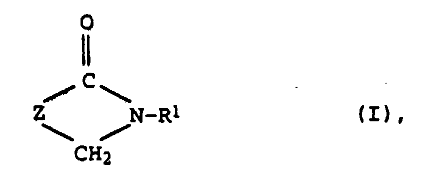 dans laquelle
Z est mis pour un groupement alkylène en C₂-C₁₀, aralkylène en C₇-C₁₂ ou phénylène ou naphtylène, et
R¹ est mis pour un groupement alkyle en C₁-C₂₀, aryle en C₆-C₁₀ ou aralkyle en C₇-C₁₂,
par la réaction hydrogénante d'un composé de formule générale II 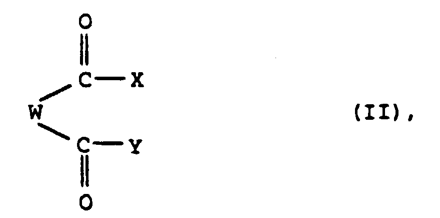 dans laquelle
W est mis pour un groupement alkylène en C₂-C₁₀, alcénylène en C₂-C₁₀, aralkylène en C₇-C₁₂ ou phénylène ou naphtylène,
et dans laquelle
X et Y forment ensemble un pont oxa ou imido de formules 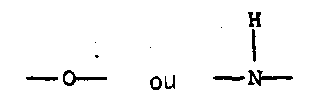 ou bien sont identiques ou différents et sont mis pour un groupement hydroxy, un groupement alcoxy en C₁-C₂₀, aryloxy en C₆-C₁₀ et/ou aralkoxy en C₇-C₁₂, et dans laquelle, si X et Y sont différents, Y peut, en plus des significations mentionnées ci-dessus, représenter un atome d'hydrogène,
sous pression élevée et à une température élevée, en présence d'un catalyseur et d'une amine, caractérisé en ce que l'on utilise comme matière de départ, des amines secondaires et/ou tertiaires de formule III
NHₙR¹ ₃₋ₙ (III)
dans laquelle le reste R¹ prend la signification susmentionnée et l'indice n est mis pour 0 ou 1, ou bien des mélanges de ces amines secondaires et/ou tertiaires avec une amine primaire de formule IV
R¹-NH₂ (IV)
la réaction étant menée avec ajout d'eau et/ou d'ammoniaque.

2. Procédé selon la revendication 1, caractérisé en ce que l'on mène la réaction en présence d'au moins 1 mole d'eau par mole d'amine utilisée.

3. Procédé selon la revendication 1, caractérisé en ce que l'on mène la réaction en présence d'au moins 1 mole d'ammoniaque par mole d'amine utilisée.

4. Procédé selon la revendication 1, caractérisé en ce que l'on mène la réaction dans un solvant.

5. Procédé selon la revendication 1, caractérisé en ce que l'on mène la réaction dans l'eau en tant que solvant.

6. Procédé selon la revendication 1, caractérisé en ce que l'on travaille à une température de 100 à 300°C et sous une pression de 50 à 350 bar.

7. Procédé selon la revendication 1, caractérisé en ce que l'on utilise un catalyseur dont le composant actif contient au moins un élément des groupes IB, VIIB ou VIII de la classification périodique des éléments.
